# EUROPEAN PATENT APPLICATION

(11) **EP 1 681 048 A1**
(43) Date of publication of application: **19.07.2006**
(21) Application number: 05000664.2
(22) Date of filing: 14.01.2005
(51) Int. Cl.: A61K 9/00, A61K 9/20

(54) **Orally disintegrating composition of olanzapine or donepezil**

(71) Applicant: Krka Tovarna Zdravil, D.D., Novo Mesto, 8501 Novo Mesto (SI)
(72) Inventor: Kroselj, Vesna, SLO-8310 Sentjernej (SI); Kolaric, Sasa, SLO-1000 Ljubljana (SI); Jakse, Renata, SLO-8220 Smarjeske Toplice (SI)
(74) Representative: UEXKÜLL & STOLBERG

(57) **Abstract**

The invention relates to orally disintegrating compositions of olanzapine or donepezil which show a very quick release of the active ingredient, as well as a process for their preparation.

## Description

The present invention relates to an orally disintegrating composition, in particular in form of tablets, which contains olanzapine or donepezil, and a process for manufacture of such a composition.

Orally disintegrating dosage forms are becoming an increasingly important issue in the area of better patient compliance comparative to the conventional solid dosage forms such as capsules and tablets for oral administration, which are the most commonly used. In particular paediatric and geriatric patients often experience difficulties in swallowing solid dosage forms. Besides, conventional solid dosage forms are not suitable for bedridden or busy and travelling patients, who may not have access to water. Thus orally dispersible tablets represent an alternative for those patients and provide for a better patient compliance with recommended pharmaceutical therapies.

Thus, various technologies have been developed which enable the preparation of compositions that disintegrate quickly in the oral cavity. These technologies are including spray drying, freeze drying, floss formation, direct compression, dry granulation, and wet granulation. However, all these technologies have their own limitations.

The spray drying technique involves spraying the drug and excipients into a chamber maintained at a high temperature. As a result this technique is not suitable for application to thermo-labile drugs. Additionally, the spray drying technology leads only to a very poor output and is very expensive.

Freeze drying on a large scale has not been found to be very effective. Moreover, it is a time consuming technique. The Zydis® tablets prepared by this technique are so fragile that the formation of the matrix material has to take place in a specific container. Tablets manufactured by this technology require a special type of packaging and careful handling during dispensing and administration to the patients, since they are prone to breakage.

The floss formation technique includes compressing microparticles of a drug and a cotton candy-like fibrous saccharide matrix, such as sucrose, dextrose, lactose and fructose. It is also known as Flash Dose technology (Fuisz) and requires specific equipment for making the specific matrix, which is sensitive to moisture, and generally results in tablets of high friability.

The wet granulation technique results in cores of a high hardness which make it difficult to obtain fast dissolving and fast disintegrating tablets. Moreover, tablets prepared thereby often lead to coarse dispersions in the oral cavity resulting in a poor patient compliance. The use of solvents and the additional drying step required in this technique may lead to a change of the polymorphic or pseudopolymorphic form of the drug or to its degradation.

The direct compression technique uses directly compressible excipients and is the most commonly used method for preparing fast disintegrating tablets. However, directly compressible excipients are not only rather expensive, but are also very coarse and granular in nature resulting in a coarse dispersion in the mouth and hence a diminished patient compliance. Using this technique makes it also very difficult to prepare fast disintegrating tablets with active ingredients having a low bulk density and poor flow properties, such as olanzapine and donepezil.

Finally, many of these techniques have proved to be successful only for specific drugs and are often not transferable to other active ingredients.

Specific orally disintegrating tablets of various active ingredients have already been described in the state of the art.

Orally disintegrating tablets containing olanzapine are known and sold under the trade name Zyprexa® VeloTab^{™}. They are prepared by a freeze-drying technique, as is for example described in EP 435 684 A1.

WO 03/103629 describes orally disintegrating tablets containing specific amounts of active ingredient having a specific particle size distribution, sprayed-dried mannitol, microcrystalline cellulose of a specific average particle size and a specific particle size distribution, and sodium croscarmellose. The tablets are prepared by a direct compression process requiring excipients, in particular a diluent like spray-dried mannitol which shows a high compressibility.

EP 1 323 417 A1 discloses tablets which are rapidly disintegrating in the oral cavity and comprise the drug, diluent and a saccharide, which has a melting point below that of the drug and the diluent. The tablets are prepared by melting and subsequently solidifying the saccharide whereupon bridges are formed between the drug and the diluent. However, the heating step to melt the saccharide requires energy and may lead to degradation of the components of the tablet and in particular of the drug. Such degradation is generally highly undesirable in case of tablets for human use.

WO 03/086361 discloses rapidly dispersing solid oral dosage compositions comprising olanzapine or ondansetron which are prepared by wet granulation or direct compression. The formulation usually also contains a filler, such as microcrystalline cellulose. However, such a filler leads, upon disintegration of the composition, to coarse particles and hence a negative sensation for the patient.

EP 1 120 109 A2 discloses highly-porous, rapidly-disintegrating and fast-dissolving dosage forms produced from steam extruded polymers. Such polymers are in particular high amylose starch or derivatized products thereof. However, it is a disadvantage that the preparation of these specific polymers requires special equipment and processes.

EP 1 260 215 A1 describes quickly disintegrating tablets which are prepared by blending an active ingredient with a saccharide and polyvinyl alcohol. The active ingredient is mixed with the saccharide, and the resulting mixture is kneaded with water including polyvinyl alcohol dissolved therein or in an organic solvent, and subjected to compression-molding. The process, however, usually involves the employing of alcohols as solvents which may result in conversion of polymorphs or formation of impurities.

In view of the deficiencies of the tablets according to the prior art, it is an object of the present invention to provide an orally disintegrating composition comprising olanzapine or donepezil, which disintegrates fast without leaving the unpleasant feeling of a coarse dispersion in the mouth and which show a low friability rendering them suitable for normal packaging and storing procedures. Further, it is an object that this composition can be prepared in a simple and cost efficient manner without the need that the active ingredients come in contact with solvents like water or is subjected to higher temperatures.

These objects are surprisingly solved by the orally disintegrating composition according to claims 1 to 9. The invention also relates to the process according to claims 10 to 11 and the tablets according to claim 12.

The orally disintegrating pharmaceutical composition according to the invention comprises
(a) olanzapine or donepezil or a salt or solvate of these as active ingredient,
(b) mannitol, and
(c) calcium silicate.

The term "orally disintegrating" means that the composition disintegrates or disperses within 90 s as measured by the in vitro disintegration test described in Ph.Eur. chapter 2.9.1. test A, page 4683, supplement 4.8 07/2004.

The composition according to the invention preferably disintegrates in less than 60 s, and more preferably in less than 30 s.

Preferably, the composition takes the form of a tablet.

The active ingredient (a) of the composition according to the invention is olanzapine or donepezil or a salt or solvate of these substances.

The olanzapine can be present in form of a salt or solvate thereof. It can also be present in form of a specific polymorph.

Examples of useful salts are the addition salts with an inorganic acid, selected from hydrochloric, hydrobromic, sulphuric, nitric, and phosphoric acid; or with an organic acid selected from acetic, propanoic, hydroxyacetic, lactic, pyruvic, oxalic, malonic, succinic, maleic, fumaric, malic, tartaric, citric, methanesulfonic, ethanesulfonic, benzenesulfonic, p-toluenesulfonic, cyclamic, salicylic, p-aminosalicylic, and pamoic acid. Examples of suitable salts are also described in EP 454 436 B1.

The olanzapine can also be present in an anhydrous form, such as those disclosed in EP 733 653 B1, which are designated therein as Form I and Form II; in US 6,348,458 designated therein as Form III, Form IV, Form V; in US 2002/0086993 A1, designated therein as Form X.

The olanzapine used may also be present in polymorphic form A or in the form of olanzapine solvates, such as the acetonitrile/methylene chloride/water, and acetonitrile/water mixed solvates, and the 2-propanol solvate, methylene chloride solvate IA, methylene chloride solvate IB as disclosed in WO 01/47933.

The most preferred polymorphic form is the polymorph I.

Also useful are the solvates disclosed in EP 733 634, and are designated therein as mono methanol solvate, mono ethanol solvate, and mono 1-propanol solvate. Also useful are hydrates of olanzapine which are disclosed e.g. in EP 831 098 B1, and are designated therein as monohydrate I and dihydrate I.

It is preferred that the composition according to the invention comprises 1 to 20, more preferably 3 to 15, and most preferred 5 to 10% by weight of olanzapine or a salt or solvate thereof.

It is also preferred that a single dosage form of the composition according to the invention, such as a tablet or capsule, comprises 1 to 25 mg of olanzapine or salt or solvate thereof, calculated as olanzapine.

The donepezil can be present in form of a salt or solvate thereof. It can also be present in form of a specific polymorph.

Examples of useful salts are the addition salts with the acids mentioned above with respect to olanzapine.

In a preferred embodiment the composition comprises donepezil hydrochloride.

Donepezil can also be used in different polymorphic or pseudopolymorphic forms, such as the polymorphic forms I to V or in its amorphous form, as is disclosed in WO 97/46527.

Preferably, the polymorphic form I is used.

It is further preferred that the composition comprises 1 to 20, more preferably 2 to 15 , and most preferred 3 to 10% by weight of donepezil or a salt or solvate thereof.

It is also preferred that a single dosage form of a composition according to the invention, such as a tablet or capsule, comprises 1 to 25 mg of donepezil or salt or solvate thereof, calculated as donepezil.

The composition according to the invention also comprises mannitol (b). In contrast to prior art formulations it is not necessary to use a highly-compressible quality, such as spray-dried mannitol.

The composition comprises preferably 30 to 90, more preferably 40 to 80 and most preferred 50 to 70 % by weight of mannitol.

The component (c) of the composition is calcium silicate. The calcium silicate can be in crystalline or amorphous form or a mixture therof. The particle size of the calcium silicate is preferably in the range from 1 to 500 µm. The average particle size is preferably 1 to 100 µm.

The composition comprises preferably 5 to 40, more preferably 10 to 30 and most preferably 12 to 25% by weight of calcium silicate.

It is surprising that the use of calcium silicate resulted in tablets having improved disintegrating properties when compared with tablets which contained disintegrants which are normally used in the preparation of rapidly dispersible compositions, such as maize starch, sodium starch glycolate, crospovidone and croscarmellose sodium. Moreover, this effect already occurred when using amounts of calcium silicate of less than 30 % by weight.

It has also surprisingly been found out that the specific combination of the mannitol (b) and the calcium silicate (c) with the active ingredients olanzapine and donepezil results in a fast disintegrating composition with a highly satisfactory mouth-feel. The rough texture which normally results when using disintegrants is avoided.

Moreover, the composition usually comprises at least one further excipient, other than mannitol and calcium silicate, selected from the group of disintegrants, binders, fillers, flavouring agents, sweetening agents, glidants, colouring agents and lubricants.

The disintegrant is preferably selected from the group consisting of crospovidone, croscarmellose sodium, sodium starch glycolate low-substituted hydroxypropyl cellulose or pregelatinized starch. Preferably the disintegrant is crospovidone or low substituted hydroxypropyl cellulose, and more preferably a combination of both is used. The low-substituted hydroxypropyl cellulose has preferably a content of hydroxypropyl groups of 7.0 to 12.9 and in particular 10.0 to 12.9 % by weight.

Examples of useful low-substituted hydroxypropyl celluloses are available under the tradenames LH-11, LH-20, LH-21, LH-22, LH-30, LH-31 and LH-32 from Shin-Etsu Chemical Co. Ltd.

It is further preferred that the composition comprises 1 to 50, more preferably 5 to 30 and most preferred 8 to 25% by weight of disintegrant.

The binders used in the composition of the invention are preferably selected from gelatin, pregelatinized starch, starch DC, acacia, tragacanth, guar gum, hydroxypropyl cellulose, low-substituted hydroxypropyl cellulose, hydroxypropylmethyl cellulose, methylcellulose, glucose, sucrose, sorbitol, polyvinyl pyrrolidone and the like. The preferred binder is low-substituted hydroxypropyl cellulose.

Preferred examples of low-substituted hydroxypropyl cellulose have been given above.

Suitable fillers are preferably selected from at least one of starch derivatives, such as corn starch, potato starch or rice starch; polysaccharides such as dextrins, maltodextrins, dextrates, microcrystalline cellulose, powdered cellulose, mixtures of microcrystalline cellulose and guar gum, coprocessed blends of microcrystalline cellulose; and polyhydric alcohols, such as xylitol and sorbitol.

Suitable sweeteners include sugars, such as sucrose, lactose and glucose; cyclamate and salts thereof; saccharin and salts thereof; and aspartame. The preferred sweetener is aspartame.

Flavouring agents can be natural or synthetic flavours such as strawberry flavour, wild cherry flavour, green apple flavour, spearmint flavour, and peppermint flavour.

Suitable lubricants are magnesium stearate, stearic acid, sodium stearyl fumarate, magnesium lauryl sulphate, polyethylene glycol, and glyceryl behenate. The preferred lubricant is magnesium stearate.

It has further proved to be advantageous if the composition according to the invention comprises granules including the mannitol (b).

In a particularly preferred embodiment, the active ingredient (a) and the calcium silicate (c) are present outside the granules which include the mannitol (b). In such an embodiment, the composition contains the active ingredient and the calcium silicate in a so-called extragranular arrangement. Such an arrangement not only offers the below-given advantages for the stability of the active ingredient, but also surprisingly results in the calcium silicate showing a higher disintegrating effect than in a granular arrangement.

The composition according to the invention is preferably prepared in a process without granulating the active ingredient.

Particularly preferred is a process comprising
(i) preparing granules including mannitol (b), and
(ii) mixing the granules with active ingredient (a) and calcium silicate (c) and
(iii) optionally molding the obtained mixture to the desired form.

In step (i) granules are prepared and this can be effected using mixing and granulating techniques customary in pharmacy, such as high shear granulation or fluidized bed granulation. Preferably, the granulation step is carried out by wet granulation using water as granulation liquid. Moreover, other excipients are usually added to the mannitol and processed to granules.

In step (ii) the granules are mixed with the active ingredient (a) and the calcium silicate (c) and this can be accomplished by conventional equipment.

In step (iii) the mixture is preferably compressed to tablets. Before compressing, a lubricant is usually added to the mixture.

This process results in tablets showing in particular the calcium silicate being outside the granules. As pointed out above, this leads to a surprisingly high disintegration effect of the calcium silicate and it is therefore assumed that the structure of the tablets differs from that of tablets prepared from granules which also contain the calcium silicate.

Therefore, the invention also relates to tablets obtainable by this preferred process.

It is a very advantageous aspect of the invention that the compositions can be prepared without the necessity that the thermo-labile and moisture sensitive compounds olanzapine and donepezil are subjected to the moisture of a wet granulation step and the elevated temperatures used in the drying procedure usually concluding the wet granulation. Moreover, as the active ingredients do not need to be subjected to wet granulation their polymorphic form remains unchanged which is a further very strong benefit of the composition according to the invention.

Further, it was found that the organoleptic properties of the composition of the invention are superior to those of compositions produced by direct compression using specific directly compressible excipients. Such excipients are very coarse and granular in nature and give only a coarse dispersion in the mouth after disintegration. This is not the case with the tablets of the present invention as they do not require a direct compression.

Moreover, the composition according to the invention has sufficient strength and only a low friability allowing for example tablets to be packed into regular containers, such as bottles, blisters, strip packs or sachets, and to be stored in bulk in drums.

The invention is now explained in more detail with reference to examples:

### EXAMPLES

### Example 1 - Orally disintegrating olanzapine tablets

| | | |
|---|---|---|
| 1 | Olanzapine | 6.7 % |
| 2 | Mannitol | 55.3 % |
| 3 | Microcrystalline cellulose | 8.0 % |
| 4 | Low-substituted HPC | 6.0 % |
| 5 | Aspartame | 0.7 % |
| 6 | Crospovidone | 8.0 % |
| 7 | Calcium silicate | 13.3 % |
| 8 | Magnesium stearate | 2 % |
| 9 | Purified water | q.s. |

| | | |
|---|---|---|
| "q.s." means "as needed" | | |

The ingredients 2 to 6 were mixed in a high shear mixer and granulated with purified water. The obtained granulate was dried and sieved and subsequently mixed with the olanzapine and the calcium silicate. Finally, magnesium stearate was admixed and the obtained mixture was compressed to tablets at < 60 N. The disintegration times of the obtained tablets were in the range of 10 to 20 s (Determined by the above given test using purified water at 37°C).

### Example 2 - Orally disintegrating olanzapine tablets

| | | |
|---|---|---|
| 1 | Olanzapine | 6.7 % |
| 2 | Mannitol | 60.0 % |
| 3 | Microcrystalline cellulose | 8.0 % |
| 4 | Povidone | 2.0 % |
| 5 | Aspartame | 0.7 % |
| 6 | Crospovidone | 8.0 % |
| 7 | Calcium silicate | 13.3 % |
| 8 | Magnesium stearate | 1.3 % |
| 9 | Purified water | q.s. |

The ingredients 2 to 6 were mixed in a high shear mixer and granulated with purified water. The obtained granulate was dried and sieved and subsequently mixed with the olanzapine and the calcium silicate. Finally, magnesium stearate was admixed and the obtained mixture was compressed to tablets at < 60 N. The disintegration times were in the range of 12 to 23 s (Determined by the above given test using purified water at 37°C) .

### Example 3 - Orally disintegrating donepezil hydrochloride tablets

| | | |
|---|---|---|
| 1 | Donepezil | 7.0 % |
| | hydrochloride | |
| 2 | Mannitol | 59.7 % |
| 3 | Microcrystalline cellulose | 8.0 % |
| 4 | Povidone | 2.0 % |
| 5 | Aspartame | 0.7 % |
| 6 | Crospovidone | 8.0 % |
| 7 | Calcium silicate | 13.3 % |
| 8 | Magnesium stearate | 1.3 % |
| 9 | Purified water | q.s. |

The ingredients 2 to 6 were mixed in a high shear mixer and granulated with purified water. The obtained granulate was dried and sieved, and subsequently mixed with donepezil and calcium silicate. Finally, magnesium stearate was admixed and the obtained mixture was compressed to tablets at < 60 N. The disintegration times of the tablets were in the range of 10 to 22 s (Determined by the above given testts using purified water at 37°C).

## Claims

1. An orally disintegrating pharmaceutical composition which comprises
(a) olanzapine or donepezil or a salt or solvate of these as active ingredient,
(b) mannitol, and
(c) calcium silicate.

2. Composition according to claim 1, which comprise donepezil hydrochloride as active ingredient.

3. Composition according to claim 1 or 2, which comprises 1 to 20, preferably 3 to 15 and most preferred 5 to 10 % by weight of olanzapine, a salt or solvate thereof.

4. Composition according to any one of claims 1 or 3, which comprises 1 to 20, preferably 2 to 15 and most preferred 3 to 10 % by weight of donepezil, a salt or solvate thereof.

5. Composition according to any one of claims 1 to 4, which comprises 30 to 90, preferably 40 to 80 and most preferred 50 to 70 % by weight of mannitol.

6. Composition according to any one of claims 1 to 5, which comprises 5 to 40, preferably 10 to 30 and most preferred 12 to 25 % by weight of calcium silicate.

7. Composition according to any one of claims 1 to 6, which comprises granules including mannitol (b).

8. Composition according to claim 7, wherein the active ingredient (a) and the calcium silicate (c) are present outside the granules.

9. Composition according to any one of claims 1 to 8, which is in form of a tablet.

10. Process for preparing the composition according to any one of claims 1 to 9 comprising
(i) preparing granules including mannitol (b), and
(ii) mixing the granules with active ingredient (a) and calcium silicate (c) and
(iii) optionally molding the obtained mixture to the desired form.

11. Process according to claim 10, wherein in step (iii) the mixture is pressed to tablets.

12. Tablets obtainable by the process according to claim 11.
